# EUROPEAN PATENT APPLICATION

(11) **EP 4 303 055 A1**
(43) Date of publication of application: **10.01.2024**
(21) Application number: 22183465.8
(22) Date of filing: 07.07.2022
(51) Int. Cl.: B60K 35/00, B60Q 3/53, B60Q 3/70

(54) **VEHICLE AND METHOD FOR REDUCING MOTION SICKNESS OF AN OCCUPANT**

(71) Applicant: Bayerische Motoren Werke Aktiengesellschaft, 80809 München (DE)
(72) Inventor: Zaki, Tarek, 81829 München (DE)

(57) **Abstract**

Disclosed are a vehicle and a method for reducing motion sickness of an occupant (1) of a vehicle (10). The method comprises the steps:
• Displaying a section of a skyview (A, B, C) on a ceiling display (2) of the vehicle (10)
• Detecting a motion of the vehicle (10), namely
- a vertical motion of the vehicle (10) and/or
- a rotation of the vehicle (10) around a horizontal axis and

• Adjusting the displayed section of the skyview (A, B, C) corresponding to the detected motion of the vehicle (10).

## Description

The present invention is related to vehicles and a method for reducing motion sickness of an occupant of the vehicle. In particular, the present invention relates to the display of artificial environmental objects.

As autonomous driving is on the rise, motion sickness is becoming an increasing issue for vehicle occupants. But not only autonomous vehicles imposes the risk of motion sickness to the occupants as is well known. Incongruent sensual perceptions (vision and acceleration) often cause motion sickness to rear-seat passengers.

For entertainment purposes, foldable ceiling displays are known from caravans and fullsize SUVs. By means of these displays, entertainment multimedia and trip information can be displayed to the occupants. For roof-mounted rear-seat entertainment displays, despite the effort to seamlessly integrate them to the glass roof of vehicles, the displays unfortunately obscure the clear view of the user to the sky. This is in particular the case when the displays are folded upwards to the ceiling.

It is an object of the present invention to overcome the drawbacks of the prior art and in particular reduce motion sickness of occupants of vehicles.

In order to solve the aforementioned problem, a basic idea of this invention is to utilize car sensors in order to display artificial content (corresponding to a sky view) on headliner-mounted displays and thereby reducing the obscurity of the displays mounted on the roof and giving the user an entertaining feature instead. Instead of using head-mounted displays, appropriate projections onto the headliner (ceiling) of the car can be used instead or in addition to matrix displays/ monitors.

In particular, a method is suggested for reducing motion sickness of an occupant (e.g. rear-seat-passenger or front-row-passenger) of a vehicle. The vehicle can be a passenger car, a truck, a van, an air plane or a vessel / ship or the like. In a first step, a section of a skyview is displayed on a ceiling display of a vehicle. The ceiling display can be a projection-based display (projector and canvas) or a light irradiating display (monitor, matrix pixel display) or the like. The skyview is the optical impression a user has when looking at the sky at the position of the vehicle. A section of the skyview is a part or a clipping of the skyview. The section can depict all elements of a certain area of the skyview (only moon and stars, for example) or can comprise all objects within the section (including all clouds, birds, planes, blue or black background). The section of the skyview is thus to be deemed at least in part an artificial phenomenon displayed on the ceiling display and/or on the ceiling liner / canvas itself. It may correspond to what the occupant would see if neither the ceiling display nor the roof or ceiling of the vehicle existed. In a next step, a motion of the vehicle, namely a vertical motion or a rotation or a super position of both is detected. This can involve height sensors (e.g. pressure sensors) and/or acceleration sensors (accelerometer and/or gyroscope and/or magnetometer and/or GPS) of the vehicle. The height profile and/or measurement can involve values of pressure sensors captured in real-time while being on the move. The same goes for further sensor-measurements described herein, like camera, optical sensors, eye-tracking, acceleration, gyroscope, magnetometer and GPS- or other positioning data. All these sensors and the data produced by these sensors may be output in real-time. The horizontal axis can be an X-axis (driving direction) or a Y-axis oriented perpendicular to the X-axis. The detected motion of the vehicle may cause motion sickness of the occupant. Thus, in a next step the displayed section of the skyview is adjusted corresponding to the detected motion of the vehicle, in particular in real time. In doing so, the skyview displayed on the ceiling display is altered such that the occupants sensual (optical) impression instantly matches the perceived motion. In other words, a relative motion of the vehicle with respect to the displayed section of the skyview concedes with the motion of the vehicle relative to an optical (real) skyview if neither the ceiling display nor the roof existed. Thus, the motion sickness of occupants of the vehicle can be reduced or prevented, at least if the displayed section of the skyview is within the view of the occupant.

The dependent claims relate to advantageous embodiments and improvements of the present invention.

Preferably, the position of the occupant within the vehicle can be determined by a sensor (in particular a camera or other optical sensor). In particular, an eye position of the user can be detected. Depending on the determined position, the real skyview of the occupant through the position of the ceiling display can be determined and a corresponding section of the skyview can be selected for display. Alternatively, the already displayed section can be adjusted in order to meet the reality.

If the ceiling display is a foldable display (foldable monitor or collapsible monitor), the section of the skyview can be altered depending on the folding state of the ceiling display. First, the folding position of the ceiling display is determined and depending on the determined folding position, in a subsequent step the displayed section of the skyview is altered or a corresponding skyview is selected for display. Thus, the foldable ceiling display can be used irrespective of the folding state for reducing or preventing motion sickness of the occupant.

Generally, the skyview at the position of the vehicle depends on several circumstances, some of which are discussed in the following: By means of a positioning sensor, the vehicle can determine its geographic position. The positioning sensor can be a sensor of the vehicle and/or a sensor of a smart device interconnected with a vehicle. By determining the geographic position, a section of the skyview corresponding to the geographic position can be selected. This promotes the correspondence of the displayed section of the skyview to the real skyview at the geographic position. The real skyview can for example be observed through an open sunroof or panoramic roof of the vehicle. Alternatively or in addition to the geographic position, a time of day can be determined and the ambient light and position of celestial bodies (e.g. the sun, the moon) can be determined and correctly displayed within the displayed section of the skyview. Alternatively or in addition to that, the weather situation can be determined via sensors of the vehicle and/or by means of weather data servers connected via the internet. Accordingly, the number of clouds or the percentage of the skyview covered by clouds can be determined and the user experience can be improved by a better match between the real skyview and the displayed section of the skyview.

The vehicle can comprise a camera, by means of which images of the real skyview can be captured and used for displaying the section of the skyview on the ceiling display. Either the entire information of the captured skyview can be displayed on the ceiling display or the position and/or the appearance of celestial bodies can be extracted and used for designing the section of the skyview before displaying same on the ceiling display. The camera can be a front camera or a dedicated skyview camera, the main optical axis of which is directed upwards or can at least show a higher inclination than the optical axis of a regular front camera of the vehicle.

In order to calibrate the vehicle for appropriately displaying the section of the skyview, in a first step, a route to be travelled by the vehicle can be determined. This can for example be derived from an explicitly or implicitly defined destination of the vehicle. For example, a navigation system can be used for estimating or receiving a current destination. Depending on a height profile and/or measurement values of pressure sensors captured during an earlier trip on the same route, the maximum and the minimum elevation of the vehicle on the route can be determined. Depending on the difference between the minimum elevation and the maximum elevation, the vehicle can associate a Zoom ratio to the maximum elevation and another Zoom ratio to the minimum elevation. As the vehicle travels on the route, depending on the current elevation of the vehicle, the section of the skyview can be adjusted for example linearly between the two predefined Zoom ratios. Thus, occupants are provided with visual information on the current elevation and deviation of elevation by merely looking at the displayed skyview section.

In order to facilitate the perception of the information regarding the current elevation, the Zoom ratio can be altered more than the effect on a real skyview would be. In other words, if a physical view at the sky from the position of the occupant would correspond to a given Zoom-in ratio if elevation of the vehicle is increased, the displayed section of the skyview can be zoomed-in by a second (and higher) Zoom-in ratio than the predefined Zoom-in ratio. Thus, although a linear relationship between elevation and Zoom ratio is neglected, feedback to the occupant is improved and easier to understand. Thus, an intuitive feedback as to the current elevation (change) is provided.

In order to provide a realistic depiction of the selected section of the skyview, the displayed section can be rotated according to an actual rotation of the vehicle around a horizontal axis and/or a vertical axis of the vehicle. Such, the change in rotational position of the vehicle exactly matches the rotation of the displayed section of the skyview and motion sickness of the occupant can be prevented in the best possible manner.

According to a second aspect of the present invention, a vehicle is proposed comprising means for reducing motion sickness of an occupant of a vehicle. The vehicle comprises a ceiling display (projector and canvas and/or monitor / matrix display). The motion detection system can use speed sensors and/or acceleration sensors. In addition, a positioning sensor system (e.g. GPS) can be used for motion detection of the vehicle. Finally, a processing unit is provided in order to evaluate signals of the motion detection system and to present a section of the skyview on the ceiling display and/or to alter the displayed section. Thus, the vehicle is capable of providing the features, a combination of features and advantages in accordance to the inventive method in detail presented above, such that further explanations can be omitted herein for the sake of brevity.

Further features and advantages of the present invention are in detail discussed in connection with the accompanying drawings.

In the drawings:
- Figure 1: is a perspective view at the interior of a prior art vehicle having a starry sky feature;
- Figure 2: is a perspective view of an interior of a first embodiment of a vehicle according to the present invention in a first state;
- Figure 3: is a perspective view of an interior of a first embodiment of a vehicle according to the present invention in a second state;
- Figure 4: a schematic diagram of a route travelled by an embodiment of a vehicle of the present invention;
- Figure 5: shows a vehicle according to an embodiment of the present invention with different folding states of a ceiling display; and
- Figure 6: shows a flow chart depicting steps of a method according to the present invention.

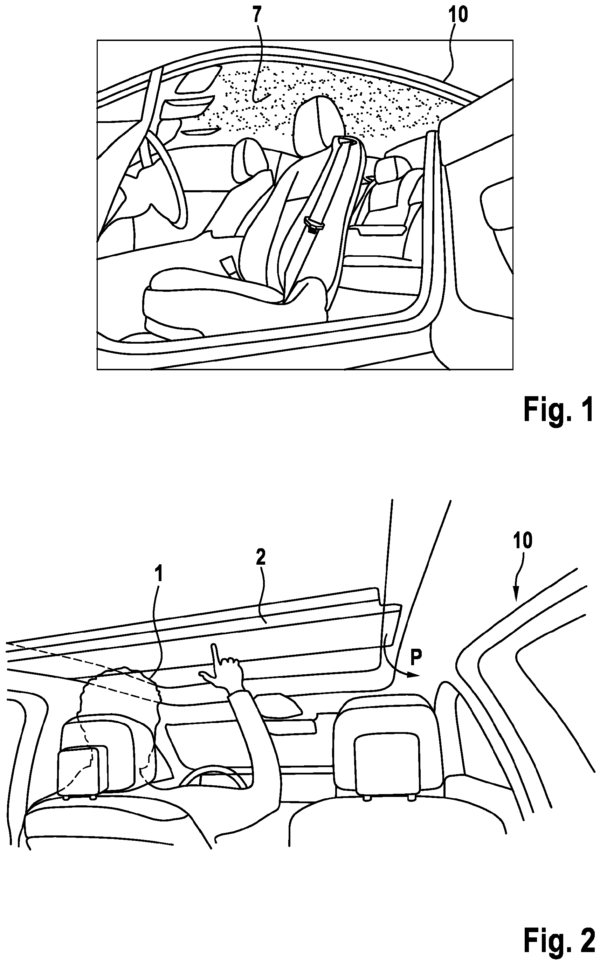

Figure 1 shows a perspective view of an interior of a prior art passenger car. The sky liner (ceiling of the car 10) comprises a plurality of single LEDs, by means of which a starry sky is imitated. The LEDs are fixedly arranged in the roof liner of the car 10. They cannot individually be switched on or off.

Figure 2 shows an occupant 1 of a vehicle 10 interacting with a ceiling display 2 which is folded away (collapsed configuration). In other words, a main surface (display surface) of the ceiling display 2 is substantially parallel to the roof liner. The occupant 1 is interacting with a touch sensitive surface of the ceiling display 2. On the ceiling display 2 a starry sky as a section of the skyview can be displayed. For unfolding the ceiling display 2, the occupant 1 is pushing a respective button in order to unfold the ceiling display 2 as is depicted by an arrow P.

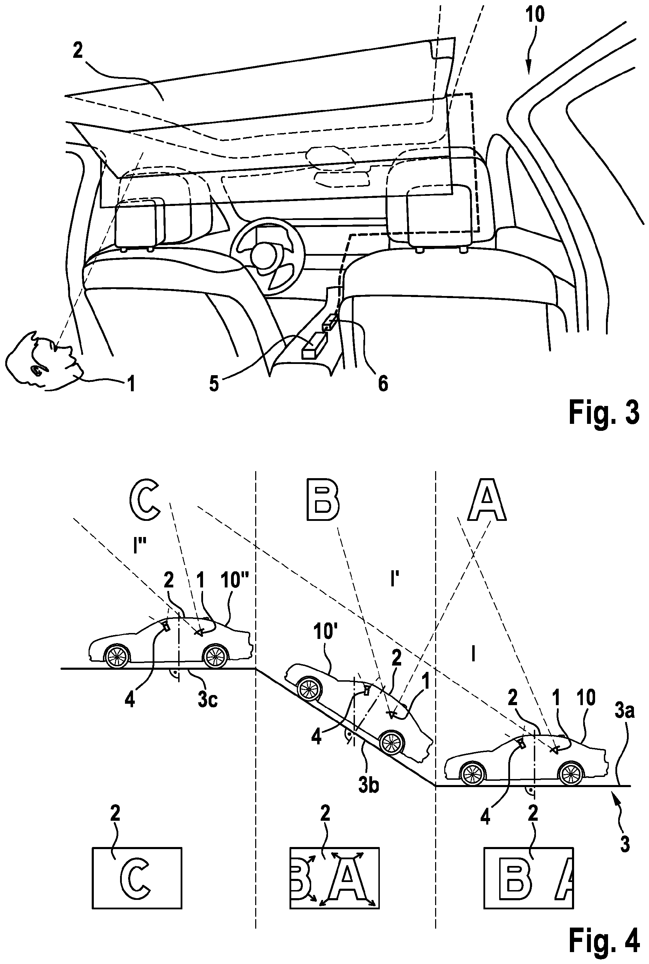

Figure 3 shows the configuration of the arrangement according to Figure 2 after unfolding the ceiling display 2. In addition, a motion detection system 5 comprising an acceleration sensor is operably connected to a processing unit 6 comprising a programmable processor for determining the motion of the vehicle. The processing unit 6 is interconnected to the ceiling monitor 2 and is adapted for interpreting signals of the motion detection system and accordingly generate a display content for displaying a section of a skyview. The skyview corresponds to a section of the real sky located behind the ceiling display 2 from the perspective of the rear seat occupant 1. Since the section of the skyview displayed on the ceiling display 2 always corresponds to the section located behind the ceiling display 2 from the perspective of the rear seat occupant 2, motion sickness of the rear seat occupant 1 is rather unlikely to occur if not fully prevented.

Figure 4 shows a vehicle 10 travelling on different sections 3a, 3b, 3c of a route 3. The vehicle 10 on route segment 3a has a lowest elevation, while a vehicle 10" on a route segment 3c has a highest elevation. In between, there is a vehicle 10' on an intermediate route segment 3b showing an intermediate elevation however being currently elevated since driving upwards. A ceiling display 2 is adapted to provide a display of a section of the skyview from the perspective of a respective rear seat occupant 1. The section of the skyview is captured by a camera 4 of the vehicle 10, 10', 10", such that from the data captured by the camera 4 an artificial skyview is presented on the ceiling display 2. Underneath each route segment 3a, 3b, 3c an exemplary display content displayed on the ceiling display 2 is depicted. The bold letters A, B, C represent celestial bodies at the respective sections 3a, 3b, 3c. Due to no elevation and no inclination, the occupant 1 in the first route segment 3a sees a relatively small depiction of letter B and a left part of letter A. After having moved on, vehicle 10' is elevated and inclined over vehicle 10. The corresponding display content on ceiling display 2 therefore has been shifted such that only a left part of letter B is depicted next to the now fully depicted letter A. Both letters are currently growing as is depicted by arrows in order to represent the ongoing elevation of the vehicle 10'. Thus, occupant 1 is provided with visual information / visual feedback on the ongoing elevation of his vehicle 10'. In route segment 3c the elevation of vehicle 10" is maximum at no inclination. Occupant 1 now merely sees a large letter C on the ceiling display 2. The lowest elevation of route segment 3a and highest elevation of route segment 3c have been known to the vehicle 10, 10', 10" due to an ongoing navigation on route 3. Depending on elevation information along route 3, the zoom ratio of the data captured by the camera 4 has been predetermined in order to adequately associate certain steps of elevation to certain zooming steps. Thus, the occupant 1 is steadily provided with visual feedback on the state of motion of vehicle 10, 10', 10".

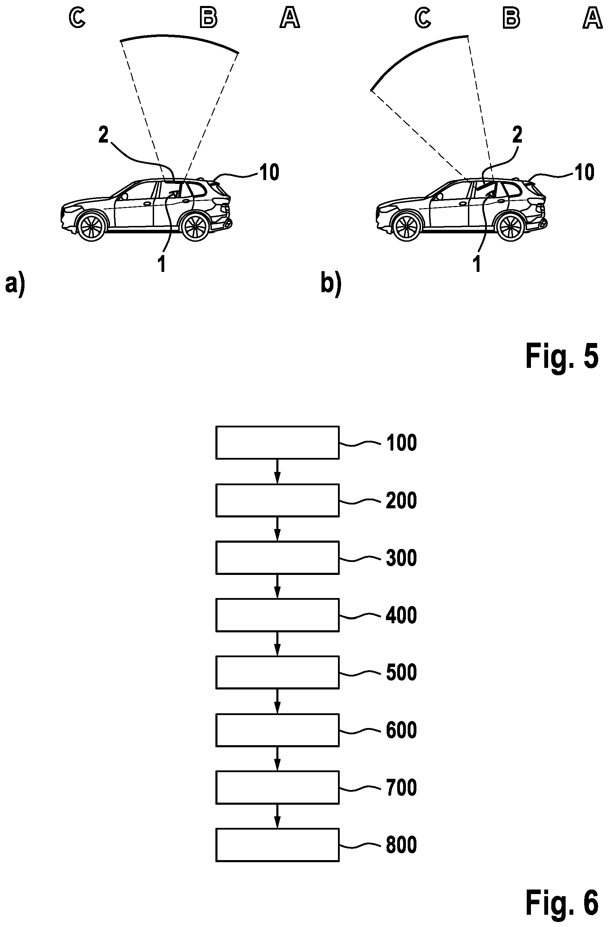

Figure 5a depicts a side view of a vehicle 10 according to an embodiment of the present invention, in which an occupant 1 is looking at a collapsed foldable ceiling display 2. Since the vehicle 10 is located underneath bold letter B and the main direction of view is vertical, the bold letter B is depicted on the collapsed ceiling display 2. After unfolding the ceiling display 2 as shown in Fig. 5b, the occupant 1 is looking at a section of the skyview being located upwards in a direction of travel of the vehicle 10. Thus, bold letter C is displayed on the unfolded ceiling display 2. Bold letters A and B are not any more displayed on the ceiling display 2.

Figure 6 shows steps of a method according to an embodiment of the present invention. In step 100, a route to be travelled by a vehicle is determined. For example, a destination of the vehicle can be estimated depending on earlier destinations travelled at corresponding day times and days of the week. Depending on this destination, a route can be calculated and selected as is well known in the prior art. At step 200 a maximum and a minimum elevation on the route is determined by means of a digital map stored in the vehicle. Depending on the maximum and the minimum elevation, in step 300 a first zoom ratio is associated to the maximum elevation and a second zoom ratio is associated to the minimum elevation. Of course, at the minimum elevation celestial bodies tend to be further away from the vehicle than at the maximum elevation. Thus, the first zoom ratio is higher than the second zoom ratio. However, in order to give a better visual feedback to the occupant as to the elevation state and elevation change of the vehicle, the first zoom ratio is picked even higher than justified by the elevation. At step 400 a section of a skyview is displayed on a ceiling display of the vehicle. The displayed section of the skyview bases on images captured by a camera of the vehicle. At step 500 a position of the occupant is determined by means of an interior camera of the vehicle. This information is used for determining a current perspective of the occupant on the ceiling display. At step 600 a folding position of the ceiling display is determined in order to determine the celestial bodies which - if the ceiling display and the roof of the vehicle would not obstruct a direct view of the occupant at the sky - would appear behind the position of the ceiling display. In step 700 a motion of the vehicle, namely a vertical motion of the vehicle and a rotation of the vehicle around both horizontal axes is detected by means of a motion sensor. Finally, at step 800 the displayed section of the skyview corresponding to the detected motion of the vehicle is adjusted. Hereby, the occupant is provided with visual feedback as to the motion state and position of the vehicle. Thus, motion signals of the occupant is reduced or prevented.

### Reference numerals:

- 1: occupant
- 2: ceiling display
- 3: route
- 3a, 3b, 3c: route segments
- 4: camera
- 5: motion detection system
- 6: processing unit
- 7: starry sky
- 10: vehicle
- 10': vehicle
- 10": vehicle
- 100 - 800: method steps
- A, B, C: celestial bodies
- P: arrow

## Claims

1. Method for reducing motion sickness of an occupant (1) of a vehicle (10) comprising the steps:
• Displaying (400) a section of a skyview (A, B, C) on a ceiling display (2) of the vehicle (10)
• Detecting (700) a motion of the vehicle (10), namely
- a vertical motion of the vehicle (10) and/or
- a rotation of the vehicle (10) around a horizontal axis and
• Adjusting (800) the displayed section of the skyview (A, B, C) corresponding to the detected motion of the vehicle (10).

2. The method of claim 1 further comprising:
- determining (500) a position of the occupant (1) and depending on the determined position,
- selecting and/or adjusting the displayed section of the skyview (A, B, C).

3. The method of claim 1 or 2 further comprising the ceiling display (2) is foldable and the method further comprises:
- determining (600) a folding position of the ceiling display (2) and depending on the determined folding position,
- selecting and/or adjusting the displayed section of the skyview (A, B, C).

4. The method of any of the preceding claims, wherein the section of the skyview (A, B, C) is selected in accordance to
- a geographic position of the vehicle and/or
- a time of day and/or
- a weather situation.

5. The method of any of the preceding claims, wherein the displayed section of the skyview (A, B, C) bases on an image captured by a camera (4) of the vehicle (10).

6. The method of any of the preceding claims further comprising:
- determining (100) a route to be travelled by the vehicle (10),
- determining (200) a maximum and a minimum elevation on the route (3) on the basis of an earlier trip on the same route and depending on the maximum and a minimum elevation
- associating (300) a first Zoom ratio to the maximum elevation and a second Zoom ratio to the minimum elevation.

7. The method of claim 6, wherein a difference between the first Zoom ratio and the second Zoom ratio is larger than needed for compensating an effect on the skyview if perceived directly.

8. The method of any of the preceding claims, wherein adjusting the displayed section of the skyview (A, B, C) corresponding to the detected motion, namely the rotation of the vehicle (10) around a horizontal axis of the vehicle, exactly reflects a detected degree of rotation.

9. Vehicle comprising means for reducing motion sickness of an occupant (1) of a vehicle (10), the means comprising:
• a ceiling display (2),
• a motion detection system (5) and
• a processing unit (6), wherein
• the ceiling display (2) is adapted for displaying a section of a skyview (A, B, C),
• the motion detection system (5) is adapted for detecting a motion of the vehicle (10), namely
- a vertical motion of the vehicle (10) and/or
- a rotation of the vehicle (10) around a horizontal axis and
• the processing unit (6) is adapted for adjusting the displayed section of the skyview (A, B, C) corresponding to the detected motion of the vehicle (10).

10. The vehicle according to claim 9 being adapted for carrying out the steps of the method according any of the claims 1 to 8.
